# EUROPÄISCHE PATENTANMELDUNG

(11) **EP 0 880 978 A2**
(43) Veröffentlichungstag der Anmeldung: **02.12.1998**
(21) Anmeldenummer: 98111600.7
(22) Anmeldetag: 18.08.1994
(51) Int. Cl.: A61M 39/10

(54) **Schlauchkupplung**

(30) Priorität: 24.08.1993 DE 4328409
(62) Teilanmeldung aus: 94112858.9
(71) Anmelder: B. BRAUN MELSUNGEN AG, 34212 Melsungen (DE)
(72) Erfinder: Rüdiger, Eick, 66459 Kirkel (DE); Heidemarie, Harms, 45143 Essen (DE); Gotthilf, Mehner, 66280 Sulzbach (DE)
(74) Vertreter: Selting, Günther, Dipl.-Ing.

(57) **Zusammenfassung**

Es wird eine Schlauchkupplung (1) zum lösbaren Verbinden eines von einer Saugvorrichtung kommenden Schlauches (3) mit einem unlösbar mit der Schlauchkupplung verbundenen Drainageschlauch (5) beschrieben, die einen auf der patientenfernen Seite (PF) angeordneten konischen Stutzen (4) aufweist, der an einem Ende eine Abschrägung (8) aufweist und zusammen mit einem auf seiner Innenseite (12) konisch ausgebildeten Außengehäuse (11) einen sich in patientenferner Richtung (PF) erweiternden Schlauchaufnahmeraum (13) bildet, wobei der Stutzen (4) in seinem patientennahen Bereich (PN) mindestens eine seitliche Abflachung (19) aufweist.

## Beschreibung

Die Erfindung bezieht sich auf eine Schlauchkupplung zum lösbaren verbinden eines von einer Saugvorrichtung kommenden Schlauches mit einem unlösbar mit der Schlauchkupplung verbundenen Drainageschlauch.

Bei der medizinischen Unterdruckdrainage besteht das Problem, den z.B. aus einer Operationswunde herausführenden Drainageschlauch lösbar mit einer Saugvorrichtung, insbesondere einer evakuierten Saugflasche (Redon-Flasche), zu verbinden. Es werden hierzu verschiedene Steckverbindungen benutzt. Bei einer bekannten Schlauchkupplung ist der Drainageschlauch fest mit einer Schlauchkupplung verbunden. Der von der Saugeinrichtung kommende Schlauch wird in einen ringspaltförmigen Aufnahmeraum eingeführt, der durch die Außenseite eines rohrförmigen Stutzens und die Innenseite eines rohrförmigen Außengehäuses gebildet wird. Nachteilig an diesem Verbinder ist die Schwierigkeit, den von der Saugeinrichtung kommenden Schlauch in den ringspaltförmigen Aufnahmeraum einzubringen und dabei auf den rohrförmigen Stutzen aufzustecken. Um die Verbindung wieder lösen zu können, sind erhebliche Kräfte erforderlich, wobei die Trennung plötzlich und ruckartig erfolgt. Hierdurch wird ein in den Schläuchen noch bestehendes Vakuum sehr plötzlich belüftet, was zum Verspritzen von möglicherweise kontaminiertem Sekret führen kann.

Aus der US-A-4 201 406 ist eine Schlauchkupplung mit einem unlösbar mit ihr verbundenen Schlauch bekannt. Diese Schlauchkupplung weist am freien Ende einen zylindrischen Stutzen mit einer Abschrägung auf, der zusammen mit einem Außengehäuse einen Schlauchaufnahmeraum bildet.

Aufgabe der vorliegenden Erfindung ist die Zurverfügungstellung einer Schlauchkupplung, die ein einfaches und sicheres Verbinden von Saug- und Drainageschlauch und ein Trennen der beiden Schläuche unter verlangsamter Aufhebung eines noch bestehenden Unterdrucks erlaubt.

Diese Aufgabe wird erfindungsgemäß mit den Merkmalen des Patentanspruchs 1 gelöst.

Die Erfindung schlägt einen auf der patientenfernen Seite angeordneten konischen Stutzen vor, der an einem Ende eine Abschrägung aufweist und zusammen mit einem auf seiner Innenseite konisch ausgebildeten Außengehäuse einen sich in patientenferner Richtung offenen Schlauchaufnahmeraum bildet, wobei der Stutzen in seinem patientennahen Bereich mindestens eine seitliche Abflachung aufweist.

Bevorzugt weist der Stutzen mehrere seitliche Abflachungen auf. Diese können regelmäßig auf den Umfang des Stutzens verteilt sein.

In einer bevorzugten Ausführungsform weist der Stutzen auf seiner Außenseite mindestens eine bis zum patientennahen Ende der Abschrägung verlaufende Nut auf, die vorzugsweise im mittleren Bereich des Stutzens beginnt.

Erfindungsgemäße Ausführungsformen können sowohl mindestens eine Nut wie auch mindestens eine seitliche Abflachung am Stutzen aufweisen.

Die erfindungsgegenständliche Schlauchkupplung wird nach den dem Fachmann geläufigen Verfahren aus den in der Medizintechnik für solche Gegenstände üblichen Materialien hergestellt.

Nachstehend wird die Erfindung anhand der Zeichnungen näher beschrieben.

Es zeigen:
- Fign. 1a-1d: verschiedene Ausführungen der Schlauchkupplung in etwa fünffacher Vergrößerung,
- Fign. 2a-2d: Querschnitte in den Ebenen II-II der Fign. 1a-1d,
- Fig. 3: einen Querschnitt in der Ebene III-III der Fig. 1c,
- Fig. 4: einen Längsschnitt durch die Schlauchkupplung in Funktionsstellung, d.h. zwei verschiedene Schläuche kuppelnd, in etwa zweifacher Vergrößerung, und
- Fig. 5: eine Darstellung entsprechend der Fig. 3, jedoch den Zustand des Entkuppelns darstellend.

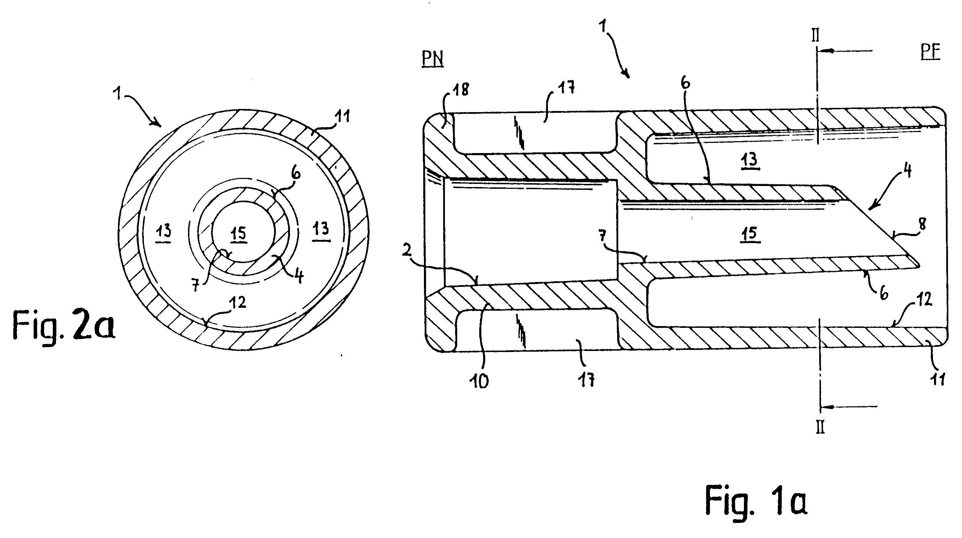

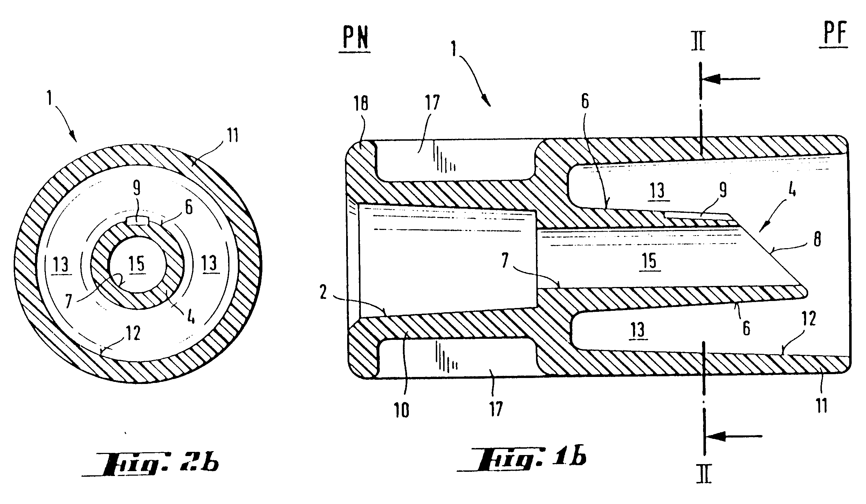

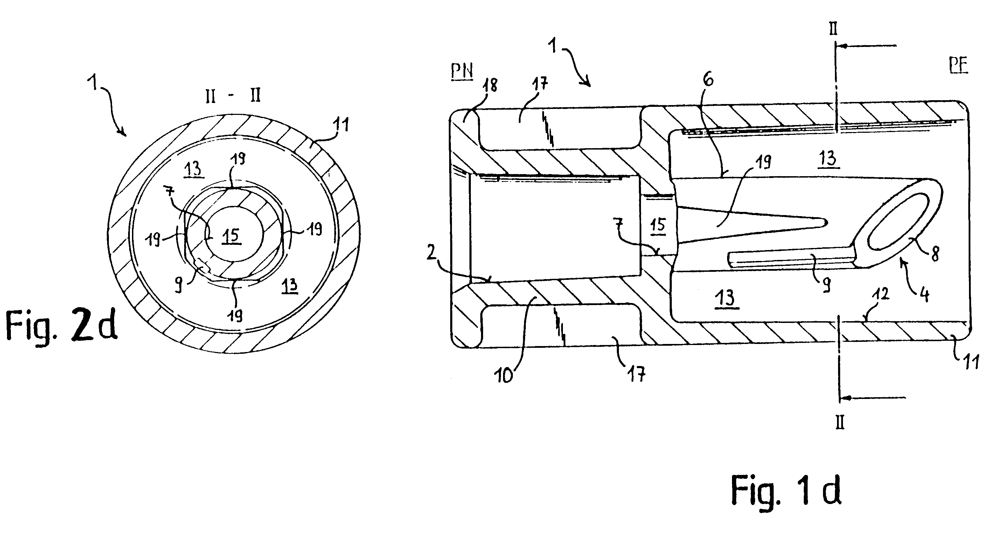

Bei den in den Fign. 1a, 1b, 2a und 2b gezeigten Schlauchkupplungen handelt es sich nicht um Ausführungsbeispiele der Erfindung.

Die Schlauchkupplung 1 weist auf ihrer patientennahen Seite PN einen Stutzen 10 mit konischer Bohrung 2 zur vorzugsweise unlösbaren Aufnahme eines Saugschlauchs 3 auf. Auf seiner patientenfernen Seite PF ist ein Anschlußstutzen 4 zur Aufnahme eines Saugschlauches 5 (Fign. 4,5) vorgesehen, welcher zu einem nicht dargestellten Auffanggefäß führt.

Der Stutzen 4 ist außen mit einem Konus 6 versehen und ist innen von einer Bohrung 7 durchsetzt, welche das Durchtritts-Lumen 15 für die abzusaugende Körperflüssigkeit bildet. Zur Erleichterung der Applikation des Schlauches 5 auf den Konus 6, bei welchem sich das Ende des Schlauches 5 aufweitet, endet der Stutzen 4 in einer Abschrägung 8.

Im Bereich des Konus 6 kann mindestens an einer Stelle eine an der Abschrägung 8 endende Nut 9 vorgesehen (Fign. 1b,4,5).

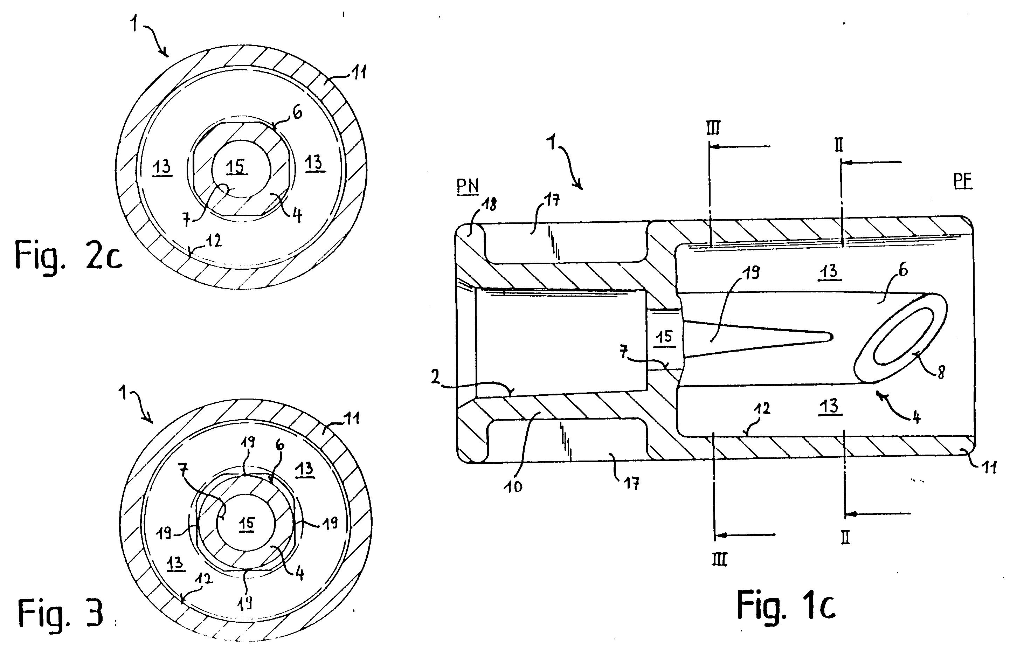

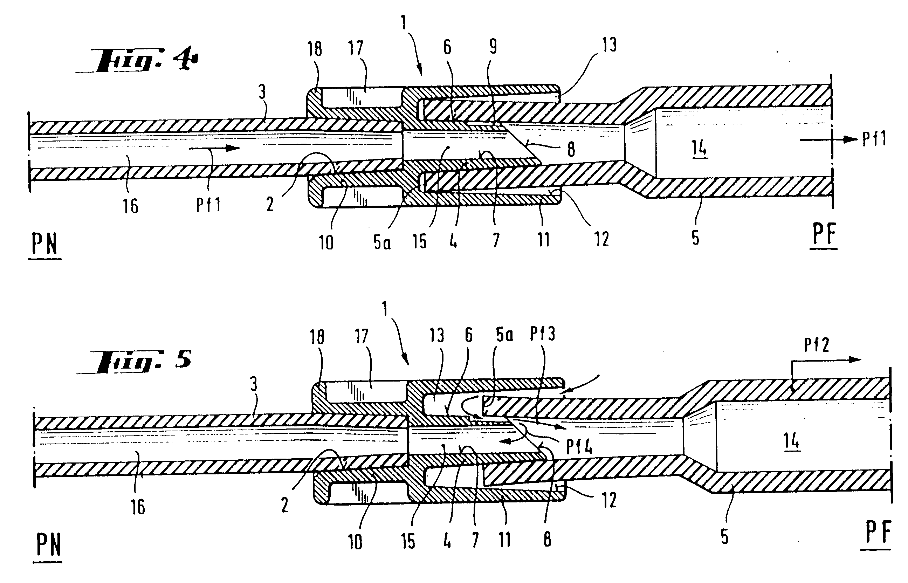

Erfindungsgemäß weist der Stutzen in seinem patientennahen Bereich seitliche Abflachungen 19 auf (Fign. 1c,2c). Diese seitlichen Abflachungen 19 sind vorzugsweise gleichmäßig über den Umfang des Stutzens verteilt, so daß sich ein Querschnitt ergibt, der dem einer Schraubenmutter ähnelt (Fign. 2c,2d,3).

In einer weiteren Ausführungsform sind sowohl seitliche Abflachungen 19 als auch mindestens eine Nut 9 vorgesehen, wobei zweckmäßigerweise die Nut 9 bzw. die Nuten 9 zwischen benachbarten Abflachungen 19 angebracht sind.

Ferner weist die Schlauchkupplung 1 ein Außengehäuse 11 auf, welches auf seiner Innenseite 12 konisch ausgebildet ist. Somit entsteht durch das Außengehäuse 11 mit Innenkonus 12 und Stutzen 4 mit Außenkonus 6 ei nin Richtung Patientenferne PF sich erweiternder Schlauchaufnahmeraum 13. Durch diese Erweiterung läßt sich der Schlauch 5 leicht in die Kupplung 1 einstecken, wie in Fig. 4 ersichtlich. Zu erkennen ist, daß sich hierbei das Schlauchende 5a des Schlauches 5 in Anpassung an die Konizität 6 des Stutzens 4 erweitert und damit eng und dichtend auf den Konus 6 des Stutzens 4 aufgeschoben ist.

Die Schlauchkupplung 1 und die beiden Schläuche 3 und 5 arbeiten nun dermaßen, als daß aus dem Körper des Patienten bzw. aus dessen Wunde Flüssigkeit in Richtung der Pfeile Pf 1 abgesaugt wird.

Soll nun, die Fig. 4 zeigt, die Schlauchverbindung 3,5 getrennt werden, so wird in Richtung des Pfeiles Pf2 der patientenferne Schlauch 5 zweckmäßigerweise unter axialem Verdrehen der Schlauchkupplung 1 gegenüber Schlauch 5 (Pf2) aus der Schlauchkupplung 1 gezogen. Dabei gleitet das Ende 5a des Schlauches 5 in Richtung PF am Konus 6 entlang. Zu Beginn des Abziehens des Schlauches 5 ist bedingt durch die dabei auftretende Schlauchkontraktion eine relativ große Zugkraft nötig. Das gegenseitige axiale Verdrehen von Schlauchkupplung 1 und Schlauch 5 erleichtert das Ablösen der Innenwand des Schlauches 5 von der Außenseite des Anschlußstutzens 4.

Das gegenseitige axiale Verdrehen wird durch die Abflachungen 19 zusätzlich erleichtert. Weiterhin begünstigen die Abflachungen 19 ein sanftes Belüften eines eventuellen Restvakuums.

Sobald das Schlauchende 5a die ebenen Seitenflächen 19 erreichen kann - wie durch Pfeile Pf3 gekennzeichnet - das bislang unter Unterdruck stehende Schlauchlumen 14 belüftet werden, und zwar in einem Maße, daß ein allmählicher Druckausgleich eingeleitet wird und somit ein sauberes, zugkraftarmes und flüssigkeitsrückstandsfreies Trennen des Schlauches 5 von der Schlauchkupplung 1 gewährleistet ist. Die allmähliche Belüftung wird zusätzlich noch durch die Abschrägung 8 am Stutzen 4 begünstigt.

Durch diese Maßnahme wird verhindert, daß eventuell vorhandene Restkörperflüssigkeit aus dem Lumen des Schlauchendes 5a herausfließt, und daß das Restvakuum in den Schläuchen 3,5 schlagartig aufgehoben wird.

Durch die einströmende Luft wird die Restflüssigkeit in Richtung PF innerhalb des Lumens 14 zum Auffangbehälter befördert und der Unterdruck allmählich abgebaut. Entsprechendes gilt für das Lumen 15 des Stutzens 4 bzw. des Lumens 16 des patientennahen Schlauches 3. Auch hier wird durch das allmähliche Einströmen von Luft in Richtung des Pfeiles Pf4 eine Befreiung von Restflüssigkeit bei der Entkopplung im Bereich der Schlauchkupplung sichergestellt.

Der körpernahe Schlauch 3 kann in der konischen Bohrung 2 des Stutzens 10 eingeklebt sein. Zur Versteifung kann das Gehäuse 11 Rippen 17 und einen Ringwulst 18 aufweisen.

## Patentansprüche

1. Schlauchkupplung (1) zum lösbaren Verbinden eines von einer Saugvorrichtung kommenden Schlauches (5) mit einem unlösbar mit der Schlauchkupplung (1) verbundenen Drainageschlauch (3) und mit einem auf der patientenfernen Seite (PF) angeordneten konischen Stutzen (4), der an einem Ende eine Abschrägung (8) aufweist und zusammen mit einem auf seiner Innenseite (12) konisch ausgebildeten Außengehäuse (11) einen in patientenferner Richtung (PF) offenen Schlauchaufnahmeraum (13) bildet,
**dadurch gekennzeichnet,**
daß der Stutzen (4) in seinem patientennahen Bereich (PN) mindestens eine seitliche Abflachung (19) aufweist.

2. Schlauchkupplung nach Anspruch 1, dadurch gekennzeichnet, daß der Stutzen (4) regelmäßig auf seinem Umfang verteilte Abflachungen (19) aufweist.

3. Schlauchkupplung nach Anspruch 1 oder 2, dadurch gekennzeichnet, daß der Stutzen (4) auf seiner Außenseite mindestens eine in patientenferner Richtung (PF) bis zum patientennahen Ende (PN) der Abschrägung (8) laufende Nut (9) aufweist.

4. Schlauchkupplung nach Anspruch 3, dadurch gekennzeichnet, daß die Nut (9) ab etwa der Mitte des konischen Stutzens (4) in patientenferne Richtung bis zum patientennahen Ende der Schräge (8) verläuft.
